# EUROPEAN PATENT APPLICATION

(11) **EP 4 285 918 A1**
(43) Date of publication of application: **06.12.2023**
(21) Application number: 21922129.8
(22) Date of filing: 27.05.2021
(51) Int. Cl.: A61K 38/12, A61K 9/14, A61K 9/19, A61K 47/02, A61P 31/04

(54) **HIGH-STABILITY DAPTOMYCIN COMPOSITION FOR INJECTION, AND PREPARATION METHOD THEREFOR AND USE THEREOF**

(30) Priority: 26.01.2021 CN 202110103108
(71) Applicant: ZHEJIANG NOVUS PHARMACEUTICALS CO., LTD., Shaoxing Zhejiang 312366 (CN); Zhejiang Medicine Co., Ltd. Xinchang Pharmaceutical Factory, Zhejiang 312599 (CN)
(72) Inventor: YUAN, Xiying, Shaoxing, Zhejiang 312366 (CN); WANG, Weibao, Shaoxing, Zhejiang 312366 (CN); WU, Guofeng, Shaoxing, Zhejiang 312366 (CN); YE, Lili, Shaoxing, Zhejiang 312366 (CN); WU, Jiali, Shaoxing, Zhejiang 312366 (CN); CAI, Jingjing, Shaoxing, Zhejiang 312366 (CN); PAN, Yibin, Shaoxing, Zhejiang 312366 (CN); ZHA, Juan, Shaoxing, Zhejiang 312366 (CN); GAO, Zhongming, Shaoxing, Zhejiang 312366 (CN); SUN, Qiming, Shaoxing, Zhejiang 312366 (CN); HE, Yimin, Shaoxing, Zhejiang 312366 (CN); WANG, Zuoliang, Shaoxing, Zhejiang 312366 (CN); PING, Jianhong, Shaoxing, Zhejiang 312366 (CN)
(74) Representative: Murgitroyd & Company
(86) International application number: PCT/CN2021/096559
(87) International publication number: WO 2022/160531

(57) **Abstract**

The present invention provides a high-stability daptomycin composition for injection and preparation method therefor and use thereof. The daptomycin composition for injection comprises daptomycin and a pharmaceutically acceptable carrier, and the pharmaceutically acceptable carrier contains a divalent metal salt, a pH regulator, and an osmotic pressure regulator; and the molar ratio of daptomycin to divalent metal salt in the daptomycin composition for injection is 1: 1.0 to 1: 3.5. The daptomycin composition for injection of the present invention significantly improves the stability of the preparation, facilitates the storage and transportation of the preparation at room temperature, and ensures the safety of drug use; And the preparation method of the present invention has advantages such as green environmental protection, suitability for industrialization.

## Description

### FIELD OF THE INVENTION

The present invention belongs to the field of pharmaceutical technology, in particular, relates to a high-stability daptomycin composition for injection, and preparation method therefor and use thereof.

### BACKGROUND OF THE INVENTION

Daptomycin (structure as shown in Formula I) is cyclic acidic lipopeptide antibiotics, which can disrupt the transport of amino acids by bacterial cell membranes, blocks the biosynthesis of teichoic acid lipids in bacterial cell wall peptidoglycan, changes the physicochemical properties of cell membranes, and makes intracellular lysates released by destroying the cell membrane of the bacteria, makes it resistant to gram-positive bacteria have rapid concentration dependent bactericidal activity against gram positive bacteria (including *methicillin resistant Staphylococcus aureus* (MRSA), *vancomycin resistant Enterococcus, glycopeptide sensitive Staphylococcus aureus, penicillin resistant Streptococcus pneumoniae, coagulase negative Staphylococcus,* etc.). It is mainly used to treat complicated skin and soft tissue infections caused by gram-positive bacteria, septicemia caused by *Staphylococcus aureus* blood infection, including right Endocarditis accompanied by Methicillin sensitive and Methicillin resistant strains.

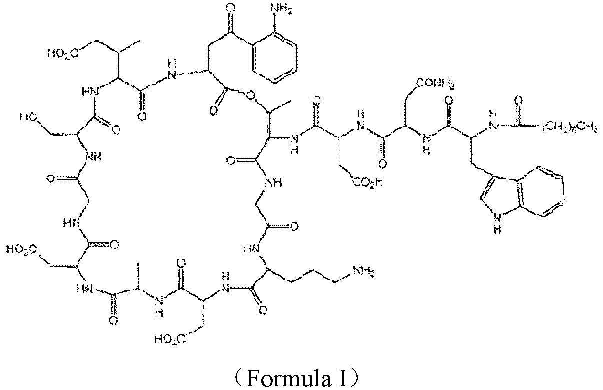

Daptomycin and its preparation solution are easy to degrade and have poor stability. The CUBICIN originally developed needs to be refrigerated (5 ± 3°C) for storage, which brings inconvenience to storage and transportation and increases costs. In order to improve the stability of daptomycin and its preparation solution, it has been studied to add protective agents (such as sucrose, sulfobutyl) to daptomycin solution-β- cyclodextrin, etc.) and/or organic solvents such as tert-butyl alcohol, However, this increases the difficulty and cost of operation, and leads to potential safety hazards in medication. Therefore, it is urgent to develop a highly stable daptomycin preparation to facilitate the storage and transportation of the preparation and ensure the effectiveness and safety of the drug.

### SUMMARY OF THE INVENTION

The object of the present invention is to provide a high-stability datomicin composition for injection. The datomicin composition comprises datomicin and a pharmaceutically acceptable carrier, the pharmaceutically acceptable carrier contains divalent metal salts and pH regulators as well as osmotic pressure regulators; wherein the molar ratio of daptomycin to the divalent metal salt in the datomicin composition for injection is 1: 1.0-1:3.5.

In the preferred technical solution of the present invention, the divalent metal salt is selected from the group consisting of calcium salts, magnesium salts and zinc salts.

In the preferred technical solution of the present invention, the divalent metal salt is selected from the group consisting of nitrate, hydrochloride, hydrobromate, hydroiodate, sulfate, gluconate, phosphate, hydrogen phosphate, and lactate.

In the preferred technical solution of the present invention, the divalent metal salt is selected from the group consisting of calcium nitrate, calcium chloride, calcium hydrobromate, calcium hydroiodate, calcium gluconate, calcium hydrogen phosphate, calcium lactate, calcium acetate, calcium phosphate, magnesium nitrate, magnesium chloride, magnesium hydrobromate, magnesium hydroiodate, magnesium sulfate, magnesium gluconate, magnesium hydrogen phosphate, magnesium lactate, magnesium acetate, magnesium phosphate, zinc nitrate, zinc chloride, zinc hydrobromate, zinc hydroiodate, zinc sulfate, zinc gluconate, zinc hydrogen phosphate, zinc lactate, zinc acetate, and zinc phosphate.

In the preferred technical solution of the present invention, the molar ratio of daptomycin to the divalent metal salt in the daptomycin composition for injection is 1:1.2-3.0, preferably 1:1.5-2.75, more preferably 1:1.75-2.50.

In the preferred technical solution of the present invention, the molar ratio of daptomycin to the hydrochloride in the daptomycin composition for injection is 1:1.2-3.0, preferably 1:1.5-2.75, more preferably 1:1.75-2.50.

In the preferred technical scheme of the present invention, the molar ratio of daptomycin to calcium chloride in the daptomycin composition for injection is 1:1.2-3.0, preferably 1:1.5-2.75, more preferably 1:1.75-2.50.

In the preferred technical solution of the present invention, the pH regulator is selected from the group consisting of sodium hydroxide, calcium hydroxide, calcium lactate, sodium lactate, sodium phosphate, disodium hydrogen phosphate, sodium dihydrogen phosphate, potassium phosphate, dipotassium hydrogen phosphate, potassium dihydrogen phosphate, citric acid, sodium citrate, sodium bicarbonate, and sodium carbonate.

In the preferred technical solution of the present invention, the pH of the daptomycin composition for injection is 3.5-6.0, preferably 4.0-5.5, more preferably 4.5-5.0.

In the preferred technical solution of the present invention, the osmotic pressure regulator is selected from the group consisting of sodium chloride, glucose, phosphate, and citrate.

In the preferred technical solution of the present invention, the molar ratio of daptomycin to the osmotic pressure regulator is 1:0-3.0, preferably 1:0.5-2.75, more preferably 1:1.0-2.5, and further preferably 1:1.5-2.25.

In the preferred technical solution of the present invention, the molar ratio of daptomycin to the divalent metal salt to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1: 1.75-2.50:1.5-2.25.

In the preferred technical solution of the present invention, the molar ratio of daptomycin to hydrochloride to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1: 1.75-2.50:1.5-2.25.

In the preferred technical solution of the present invention, the molar ratio of daptomycin to calcium chloride to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1:1.75-2.50:1.5-2.25.

In the preferred technical solution of the present invention, the molar ratio of daptomycin to calcium chloride to sodium chloride in the daptomycin composition for injection is 1:1.2:2.74 and to adjust the pH of the daptomycin composition for injection to 4.0 by sodium hydroxide.

In the preferred technical solution of the present invention, the molar ratio of daptomycin to calcium chloride to sodium chloride in the daptomycin composition for injection is 1:1.2:2.74 and to adjust the pH of the daptomycin composition for injection to 5.0 by sodium hydroxide.

In the preferred technical solution of the present invention, the molar ratio of daptomycin to calcium chloride to sodium chloride in the daptomycin composition for injection is 1:1.2:2.74 and to adjust the pH of the daptomycin composition for injection to 6.0 by sodium hydroxide.

In the preferred technical solution of the present invention, the molar ratio of daptomycin to calcium chloride to sodium chloride in the daptomycin composition for injection is 1:1.5:2.5 and to adjust the pH of the daptomycin composition for injection to 4.5 by sodium hydroxide.

In the preferred technical solution of the present invention, the molar ratio of daptomycin to calcium chloride to sodium chloride in the daptomycin composition for injection is 1:1.75:2.1 and to adjust the pH of the daptomycin composition for injection to 5.0 by sodium hydroxide.

In the preferred technical solution of the present invention, the molar ratio of daptomycin to calcium chloride to sodium chloride in the daptomycin composition for injection is 1:2.5:1.1 and to adjust the pH of the daptomycin composition for injection to 5.0 by sodium hydroxide.

In the preferred technical solution of the present invention, the molar ratio of daptomycin to calcium chloride in the daptomycin composition for injection is 1:3.5, and to adjust the pH of the daptomycin composition for injection to 5.0 by sodium hydroxide.

In the preferred technical solution of the present invention, the daptomycin composition for injection is selected from freeze-drying powder or spray-drying powder.

In the preferred technical solution of the present invention, the total impurity content in the daptomycin composition for injection is ≤ 7.0%, and the content of the dehydrated daptomycin is ≤ 4.0%, the content of the β-isomer is ≤ 1.5%, the content of the lactone hydrolysate is ≤ 1.5%.

In the preferred technical solution of the present invention, the total impurity content in the daptomycin composition for injection is ≤ 6.0%, and the content of the dehydrated daptomycin is ≤ 3.5%, the content of the β-isomer is ≤ 1.0%, the content of the lactone hydrolysate≤ 1.0%.

In the preferred technical solution of the present invention, the total impurity content in the daptomycin composition for injection is ≤ 5.0%, and the content of the dehydrated daptomycin is ≤ 3.0%, the content of the β-isomer is ≤ 0.8%, the content of the lactone hydrolysate is ≤ 0.8%.

In the preferred technical solution of the present invention, the total impurity content in the daptomycin composition for injection is ≤ 4.0%, and the content of dehydrated daptomycin is ≤ 2.0%, the content of the β-isomer is ≤ 0.6%, the content of the lactone hydrolysate is ≤ 0.6%.

In the preferred technical solution of the present invention, the total impurity content in the daptomycin composition for injection is ≤ 3.0%, and the content of dehydrated daptomycin is ≤ 1.0%, the content of the β-isomer is ≤ 0.5%, the content of the lactone hydrolysate is ≤ 0.5%.

In the preferred technical solution of the present invention, the water content of the daptomycin composition for injection is ≤ 5.0%, preferably ≤ 4.0%, and more preferably ≤ 3.0%.

In the preferred technical solution of the present invention, the clarity of the solution after re-dissolution of the daptomycin composition for injection is less than or close to the turbidity standard solution No.1.

In the preferred technical solution of the present invention, the solution absorbance A₄₅₀ₙₘ after re-dissolution of the daptomycin composition for injection is ≤0.50, preferably A₄₅₀ₙₘ ≤ 0.40, more preferably A₄₅₀ₙₘ ≤ 0.30.

Another object of the present invention is to provide a preparation method for high-stability datomycin composition for injection, the datomycin composition for injection comprises datomycin and a pharmaceutically acceptable carrier, the pharmaceutically acceptable carrier contains divalent metal salts, pH regulators, and osmotic pressure regulators; wherein the molar ratio of daptomycin to the divalent metal salt in the daptomycin composition for injection is 1:1.0-1:3.5; the method comprises the following steps: dissolving a required amount of daptomycin and a divalent metal salt in water for injection to form an aqueous solution, adding a pH regulator to the aqueous solution, and then adjusting a pH of the aqueous solution to 3.5-6.0, optionally adding a required amount of the osmotic pressure regulator to adjust the aqueous solution to isotonic, filtering to obtain a filtrate , and then treating the filtrate by freeze-drying or spray-drying to obtain a datomycin composition for injection.

In the preferred technical solution of the present invention, the filtration is a filter membrane filtration, preferably an aperture of filter membrane is ≤ 0.45µm, more preferably 0.40µm, 0.35µm, 0.30µm, 0.25µm, or 0.22µm.

In the preferred technical solution of the present invention, the divalent metal salt is selected from the group consisting of of calcium salts, magnesium salts, and zinc salts.

In the preferred technical solution of the present invention, the divalent metal salt is selected from the group conisisting of nitrate, hydrochloride, hydrobromate, hydroiodate, sulfate, gluconate, phosphate, hydrogen phosphate, and lactate.

In the preferred technical solution of the present invention, the divalent metal salt is selected from the group consisting of calcium nitrate, calcium chloride, calcium hydrobromate, calcium hydroiodate, calcium gluconate, calcium hydrogen phosphate, calcium lactate, calcium acetate, calcium phosphate, magnesium nitrate, magnesium chloride, magnesium hydrobromate, magnesium hydroiodate, magnesium sulfate, magnesium gluconate, magnesium hydrogen phosphate, magnesium lactate, magnesium acetate, magnesium phosphate, zinc nitrate, zinc chloride, zinc hydrobromate, zinc hydroiodate, zinc sulfate, zinc gluconate, zinc hydrogen phosphate, zinc lactate, zinc acetate, and zinc phosphate.

In the preferred technical solution of the present invention, the molar ratio of daptomycin to the divalent metal salt in the daptomycin composition for injection is 1:1.2-3.0, preferably 1:1.5-2.75, more preferably 1:1.75-2.50.

In the preferred technical solution of the present invention, the molar ratio of daptomycin to hydrochloride in the daptomycin composition for injection is 1:1.2-3.0, preferably 1:1.5-2.75, more preferably 1:1.75-2.50.

In the preferred technical scheme of the present invention, the molar ratio of daptomycin to calcium chloride in the daptomycin composition for injection is 1:1.2-3.0, preferably 1:1.5-2.75, more preferably 1:1.75-2.50.

In the preferred technical solution of the present invention, the pH regulator is selected from the group consisting of sodium hydroxide, calcium hydroxide, calcium lactate, sodium lactate, sodium phosphate, disodium hydrogen phosphate, sodium dihydrogen phosphate, potassium phosphate, dipotassium hydrogen phosphate, potassium dihydrogen phosphate, citric acid, sodium citrate, sodium bicarbonate, and sodium carbonate.

In the preferred technical solution of the present invention, the pH of the daptomycin composition for injection is 3.5-6.0, preferably 4.0-5.5, more preferably 4.5-5.0.

In the preferred technical solution of the present invention, the osmotic pressure regulator is selected from the group consisting of sodium chloride, glucose, phosphate, and citrate.

In the preferred technical solution of the present invention, the molar ratio of daptomycin to the osmotic pressure regulator is 1:0-3.0, preferably is 1:0.5-2.75, more preferably is 1:1.0-2.5, and further preferably is 1:1.5-2.25.

In the preferred technical solution of the present invention, the molar ratio of daptomycin to the divalent metal salt to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1: 1.75-2.50:1.5-2.25.

In the preferred technical solution of the present invention, the molar ratio of daptomycin to hydrochloride to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1: 1.75-2.50:1.5-2.25.

In the preferred technical solution of the present invention, the molar ratio of daptomycin to calcium chloride to the osmotic pressure regulator in the daptomycin composition for injection is 1:1.0-3.5:0-3.0, preferably 1:1.2-3.0:0.5-2.75, more preferably 1:1.5-2.75:1.0-2.5, and further preferably 1:1.75-2.50:1.5-2.25.

In the preferred technical solution of the present invention, the molar ratio of daptomycin to calcium chloride to sodium chloride in the daptomycin composition for injection is 1:1.2:2.74 and to adjust the pH of the composition to 4.0 by sodium hydroxide.

In the preferred technical solution of the present invention, the molar ratio of daptomycin to calcium chloride to sodium chloride in the daptomycin composition for injection is 1:1.2:2.74 and to adjust the pH of the daptomycin composition for injection to 5.0 by sodium hydroxide.

In the preferred technical solution of the present invention, the molar ratio of daptomycin to calcium chloride to sodium chloride in the daptomycin composition for injection is 1:1.2:2.74 and to adjust the pH of the composition to 6.0 by sodium hydroxide.

In the preferred technical solution of the present invention, the molar ratio of daptomycin to calcium chloride to sodium chloride in the daptomycin composition for injection is 1:1.5:2.5 and to adjust the pH of the daptomycin composition for injection to 4.5 by sodium hydroxide.

In the preferred technical solution of the present invention, the molar ratio of daptomycin to calcium chloride to sodium chloride in daptomycin composition for injection is 1:1.75:2.1 and to adjust the pH of the daptomycin composition for injection to 5.0 by sodium hydroxide.

In the preferred technical solution of the present invention, the molar ratio of daptomycin to calcium chloride to sodium chloride in the daptomycin composition for injection is 1:2.5:1.1 and to adjust the pH of the daptomycin composition for injection to 5.0 by sodium hydroxide.

In the preferred technical solution of the present invention, the molar ratio of daptomycin to calcium chloride in the daptomycin composition for injection is 1:3.5, and to adjust the pH of the daptomycin composition for injection to 5.0 by sodium hydroxide.

In the preferred technical solution of the present invention, the daptomycin composition for injection is selected from the group consisting of freeze-drying powders or spray-drying powders.

In the preferred technical solution of the present invention, the freeze drying method comprises the following steps:
1) filling the filtrate into a penicillin bottle, and then placing it in a freeze-drying oven, cooling to -20°C~ -70°C to make the filtrate frozen completely;
2) heating up to -10°C-5°C, and then maintaining a vacuum of 0-0.25 mbar, and drying;
3) heating up to 5°C~40°C, and drying to obtain a datomycin composition.

In the preferred technical solution of the present invention, the spray drying method comprises the following steps: setting the inlet air temperature to 150-230, the outlet air temperature to 80-150°C, the process gas flow at 25-45kg/h, and the nozzle gas flow at 3-5.5kg/h; after these parameters are stabilized, spray-drying the aqueous solution of daptomycin to obtain a datomycin composition.

In the preferred technical solution of the present invention, the inlet air temperature is 180-220°C, preferably 200-210°C.

In the preferred technical solution of the present invention, the outlet air temperature is 90-130°C, preferably 100-110°C.

In the preferred technical solution of the present invention, the process gas flow is 30-43kg/h, preferably 35-40kg/h.

In the preferred technical solution of the present invention, the nozzle gas flow is 3.5-5.0kg/h, preferably 4.0-4.5kg/h.

In the preferred technical solution of the present invention, the total impurity content in the daptomycin composition for injection is ≤ 7.0%, and the content of the dehydrated daptomycin is ≤ 4.0%, the content of β-Isomer is ≤ 1.5%, the content of the lactone hydrolysate is ≤ 1.5%.

In the preferred technical solution of the present invention, the total impurity content in the daptomycin composition for injection is ≤ 6.0%, and the content of the dehydrated daptomycin is ≤ 3.5%, the content of β-Isomer is ≤ 1.0%, the content of the lactone hydrolysate is ≤ 1.0%.

In the preferred technical solution of the present invention, the total impurity content in the daptomycin composition for injection is ≤ 5.0%, and the content of then dehydrated daptomycin is ≤ 3.0%, the content of β-Isomer is ≤ 0.8%, the content of the lactone hydrolysate is ≤ 0.8%.

In the preferred technical solution of the present invention, the total impurity content in the daptomycin composition for injection is ≤ 4.0%, and the content of the dehydrated daptomycin is ≤ 2.0%, the content of β-Isomer is ≤ 0.6%, the content of the lactone hydrolysate is ≤ 0.6%.

In the preferred technical solution of the present invention, the total impurity content in the daptomycin composition for injection is ≤ 3.0%, and the content of the dehydrated daptomycin is ≤ 1.0%, the content of β-Isomer is ≤ 0.5%, the content of the lactone hydrolysate is ≤ 0.5%.

In the preferred technical solution of the present invention, the water content of the daptomycin composition for injection is ≤ 5.0%, preferably ≤ 4.0%, and more preferably ≤ 3.0%.

In the preferred technical solution of the present invention, the clarity of the solution after re-dissolution of the daptomycin composition for injection is less than or close to the turbidity standard solution No. 1.

In the preferred technical solution of the present invention, the solution absorbance A₄₅₀ₙₘ after re-dissolution of the daptomycin composition for injection is ≤0.50, preferably A₄₅₀ₙₘ ≤ 0.40, more preferably A₄₅₀ₙₘ ≤ 0.30.

Another object of the present invention is to provide a use of a high-stability datomicin composition for injection in the preparation for antibacterial drugs.

In the preferred technical solution of the present invention, the datomicin composition is selected from freeze-drying powders or spray-drying powders.

In the preferred technical solution of the present invention, the datomicin composition is used for treating infections caused by Gram-positive bacteria.

In the preferred technical solution of the present invention, the infection is selected from the group consisting of skin and soft tissue infections, endocarditis, bacteremia, or complications thereof.

In the preferred technical solution of the present invention, the infection is selected from the group consisting of complex skin infection and soft tissue infection (cSSTIs), right ventricular endocarditis (RIE) caused by Staphylococcus aureus, bacteremia, or complications thereof.

In the preferred technical solution of the present invention, the datomicin composition further comprises other antibacterial drugs.

In the preferred technical solution of the present invention, other antibacterial drugs are selected from the group consisting of quinolones, penicillin, cephalosporins, β-lactams, aminoglycosides, macrolides, and lincomycin drugs.

In the preferred technical solution of the present invention, the quinolone drugs are selected from the group consisting of norfloxacin (norfloxacin), enoxacin, ciprofloxacin, ofloxacin, levofloxacin, pefloxacin, and sparfloxacin.

In the preferred technical solution of the present invention, the penicillin drugs are selected from the group consisting of penicillin G, penicillin V, methicillin (Xinqing I), ampicillin (Ampicillin), amoxicillin (hydroxy ampicillin), ticarcillin (carboxythiophene penicillin), piperacillin (oxypiperazine penicillin).

In the preferred technical solution of the present invention, the cephalosporins drugs are selected from the group consisting of Cefazolin, cefradine, Cefalexin, cefradine, Cefadroxil, Cefuroxime (cefuroxime), Cefamandole, cefoxitin, cefmetazole, Cefuroxime axetil, Cefaclor, Cefotaxime, ceftazidine, Ceftriaxone, Cefoperazone, Ceftizoxime, Cefodizime, Cefixime, cefpodoxime lipid, Cefteram Pivoxil, Cefetamet Pivoxil, Cefepime, Cefpirome.

In the preferred technical solution of the present invention, the β-lactam drugs are selected from the group consisting of imipenem, sitatin, and meropenem.

In the preferred technical solution of the present invention, the aminoglycoside drug is selected from the group consisting of gentamicin, tobramycin, netilmicin, amikacin (amikacin), streptomycin, and spectinomycin.

In the preferred technical solution of the present invention, the macrolide drugs are selected from the group consisting of erythromycin, methyl erythromycin (clarithromycin), roxithromycin, azithromycin, j osamycin, midecamycin, spiramycin.

In the preferred technical solution of the present invention, the lincomycin drugs are selected from the group consisting of lincomycin and clindamycin.

The freeze-drying machine of the present invention adopts a vacuum freeze-drying machine with model Lyo1 (CIP) from Shanghai Dongfulong Technology Co., Ltd.

The spray-drying machine of the present invention adopts the spray dryer MS35 of Spike Company (SPX).

Unless otherwise specified, when the present invention relates to the percentage between liquid and liquid, the said percentage is volume/volume percentage. When the present invention relates to the percentage between liquid and solid, the percentage is the volume/weight percentage; When the present invention relates to the percentage between solid and liquid, the percentage is the weight/volume percentage. The rest is weight/weight percentage.

In comparison with the prior art, the present invention has the following beneficial effects: 1)The present invention scientifically screens the components and proportion of the composition of daptomycin for injection, significantly improves the stability of the preparation, overcomes the problems of unqualified clarity and excessive osmotic pressure caused by high concentration of calcium ions, facilitates the storage and transportation of the preparation at room temperature, and ensures the safety of drug use. 2) The daptomycin composition for injection of the invention has the advantages of simple composition, no protective agent and organic solvent, good stability, low price and easy availability of auxiliary materials, better cost, controllable product quality, green environmental protection, suitable for industrialization.

### DESCRIPTION OF THE PREFERRED EMBODIMENTS

Hereinafter, the present invention will be further described with reference to the embodiments. The embodiments of the present invention are only used to illustrate the technical solutions of the present invention, and do not limit the present invention.

### Example 1: Preparation of Datomycin Composition for Injection

The preparation method of datomicin composition for injection comprises the following steps:
1. Take 500 g of daptomycin and 51.4 g of calcium chloride, dissolve them to 2.5 L of water for injection, then add a sodium hydroxide solution to adjust pH 4.5, and then add 45.1 g of sodium chloride to obtain a solution, afterwards filter the solution through 0.22 µM membrane filtration to collect a filtrate;
2. Set an inlet air temperature of the spray dryer to 200°C, an outlet air temperature to 110°C, a process gas flow to 35kg/h, and a nozzle gas flow to 4.5kg/h; then dry the filtrate collected through a spray dryer to obtain spray drying powders. The spray drying powders are divided into vials, stoppered, capped and sealed. Take samples and then test the water content of 3.0%.

### Example 2: Preparation of Datomycin Composition for Injection

The preparation method of datomicin composition for injection comprises the following steps:
Take 500 g of daptomycin and 60 g of calcium chloride, dissolve them to 1.5 L of water for injection, then add a sodium hydroxide solution to adjust pH 5.0, and then add 38 g of sodium chloride to obtain a solution, afterwards filter the solution through 0.22 µM membrane filtration, to collect a filtrate, the filtrate is divided into vials. After half stoppering, place the vials with the filtrate to a freeze-drying oven to pre-frozen for about 4 hours below -40°C. And then heat up and control a temperature below 5°C, control a vacuum degree around 0.25 mbar, and control a sublimation drying for 20 hours. The temperature is programme controlled to ensure to make a desorption dry for about 7 hours under the temperature is not higher than 25°C. Fill with nitrogen, press a stopper, and take out of the freeze-drying oven and roll a lid. Take samples and test for a water content of 2.0%.

### Test Example 1: Study on Stability of Datomycin Composition for Injection

The datomicin composition for injection prepared by Examples 1 and 2 and the original commercially available formulations CUBICIN RF and CUBICIN are placed at 40°C for one month to detect their related substances and changes. The results are shown in Table 1.

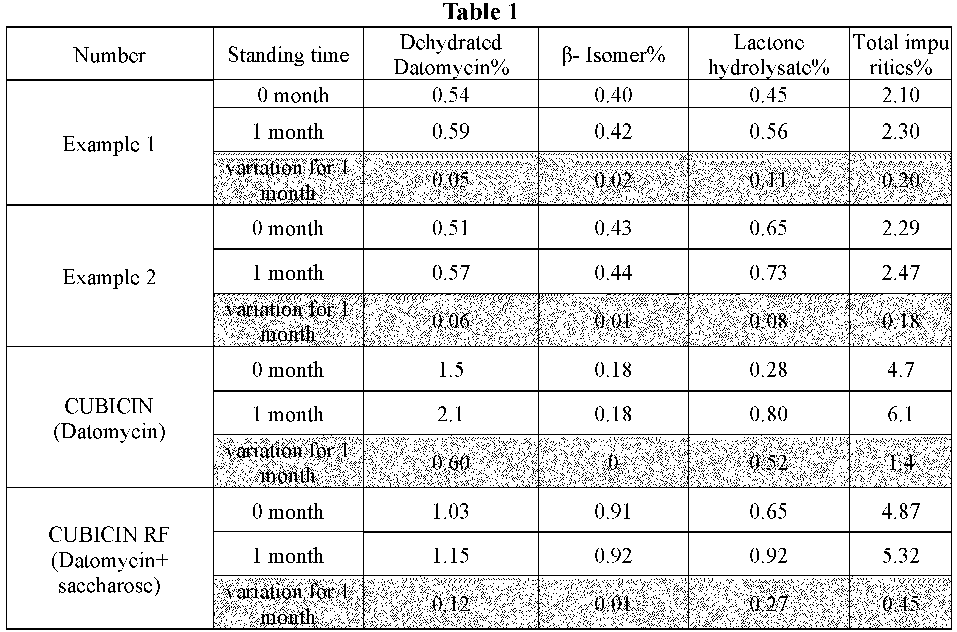

The results shows that the stability of the spray drying powders or the freeze drying powders is better than that of CUBICIN and CUBICIN RF.

### Test Example 2: Study on the Resolubility Stability of Daptomycin Composition for Injection

Add the daptomycin composition for injection prepared by Examples 1 and 2 and CUBICIN to a water for injection, dissolve them to form a 50 mg/ml solution, and then place the solution at 2-8°C for 48 hours to investigate the changes of related substances. The results are shown in Table 2.

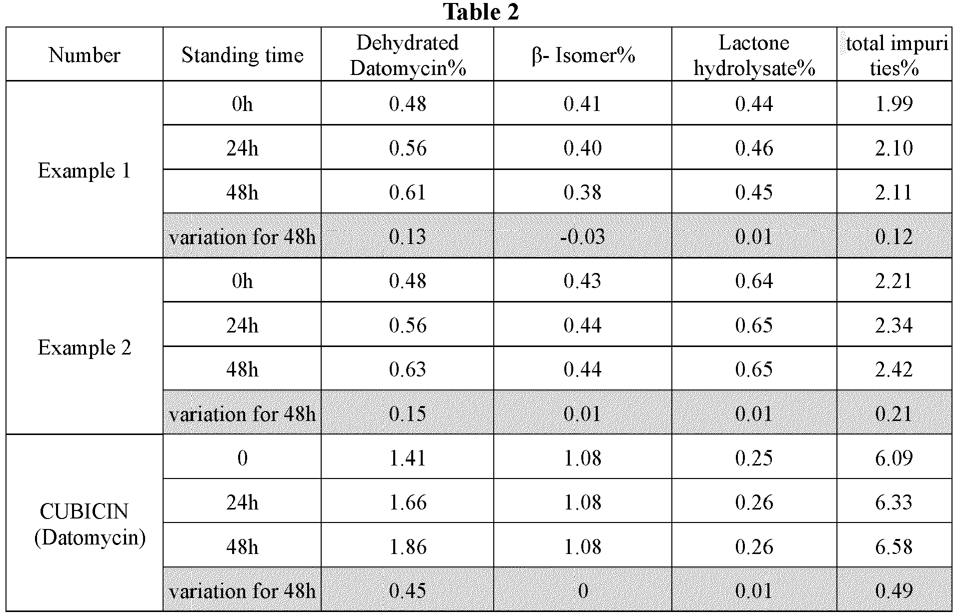

The results shows that in comparison with CUBICIN, the spray-drying powders of Example 1 and the freeze-drying powders of Example 2 have better stability than that of CUBICIN , so as to ensure the drug safety of patients.

### Test Example 3: Study on the Stability of Datomycin Calcium Chloride Solution

Dissolve 5.0 g of daptomycin and 1.2 g of calcium chloride to 100ml of water for injection, evenly divide them into three parts, then add a sodium hydroxide solution to the three parts to respectively adjust to pH 4.0, pH 5.0, and pH 7.0; and then filter to obtain a filtrate, and divide the filtrate into vials, seal them, and then place them at 25 °C for 24, in order to observe the changes of relevant substances. The results are shown in Table 3.

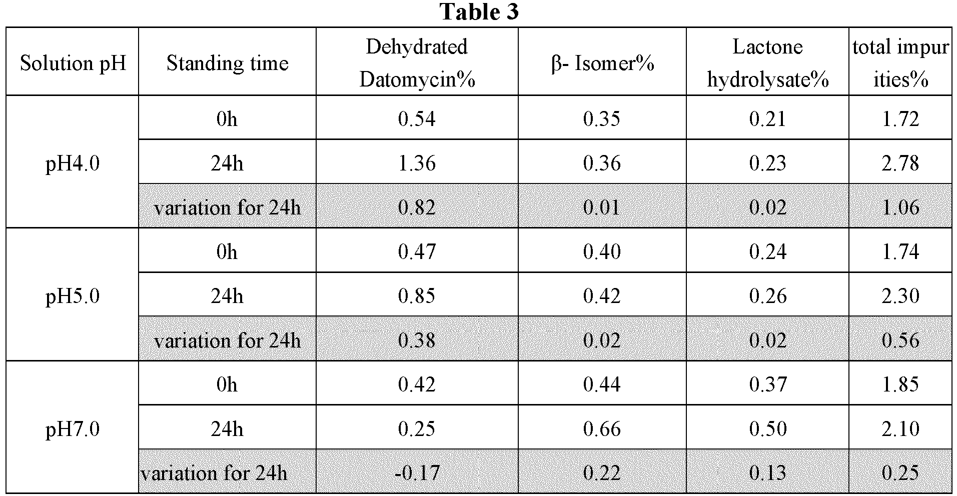

The results shows that with the increase of pH value, the change degree of related substances of daptomycin becomes smaller, and the stability is better when the pH is high_{∘}

### Example 3: Preparation of Datomycin Composition for Injection

Take 500g of daptomycin and 41g of calcium chloride, dissolve them to 2.5 L of water for injection, then add a sodium hydroxide solution to adjust pH 4.0, and then add 48g of sodium chloride, filter, and collect a filtrate. Set an inlet air temperature at 220°C, an outlet air temperature at 120°C, a process gas flow at 30kg/h, a nozzle gas flow at 3.5kg/h, and then collect the filtrate for spray-drying to obtain spray drying powders, afterwards separately pack the spray drying powders into vials, stoppered, capped and sealed.

### Examples 4-6: Preparation of Datomycin Composition for Injection

According to the method and the composition prepared by Example 3, respectively adjust the pH values of the daptomycin solution of Examples 4-6 to pH5.0, pH6.0 and pH7.0 with a sodium hydroxide solution; after spray-drying, obtain spray drying powders of daptomycin for injection.

### Test Example 4: Stability Study of Datomycin Composition for Injection

The spray drying powders prepared by Examples 3-6 are placed at 60°C for 10 days to investigate the changes of related substances. The results are shown in Table 4.

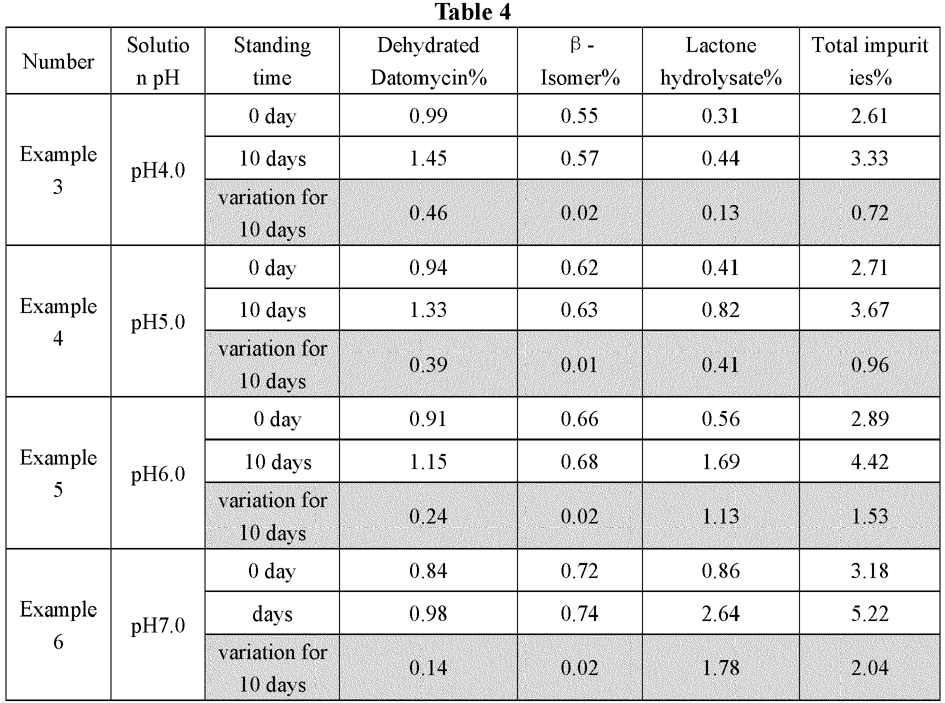

The results shows that with the increase of pH value, the stability of spray drying powder decreases, and the change range of total impurities increases. The data shows that the stability of daptomycin for injection is better when the pH is low in the presence of solid powder.

### Example 7: Preparation of Datomycin Composition for Injection

Take 500 g of daptomycin and 41 g of calcium chloride, dissolve them to 2.5 L of water for injection, then add a sodium hydroxide solution to adjust pH 5.0, and then add 48g of sodium chloride, filter, and collect a filtrate. Set an inlet air temperature at 220°C, an outlet air temperature at 120°C, a gas flow at 30kg/h, a nozzle gas flow at 3.5kg/h, collect the filtrate for spray drying to obtain spray drying powders, and then separately pack the spray drying powders into vials, stoppered, capped and sealed.

### Example 8: Preparation of Datomycin Composition for Injection

Take 500 g of daptomycin and 41 g of calcium chloride, dissolve them to 2.5 L of water for injection, then add a sodium hydroxide solution to adjust pH 7.0, and then add 48g of sodium chloride, filter, and collect a filtrate. Set an inlet air temperature at 220°C, an outlet air temperature at 120°C, a process gas flow at 30 kg/h, a nozzle gas flow at 3.5kg/h; collect the filtrate for spray-drying to obtain spray drying powders, and then separately pack the spray drying powders into vials, stoppered, capped and sealed.

### Test Example 5: Study on Stability of Datomycin Composition for Injection

The spray drying powders prepared by Examples 7 and 8 and CUBICIN RF and CUBICIN are placed at 40°C for two months to detect their related impurities and changes. The results are shown in Table 1.

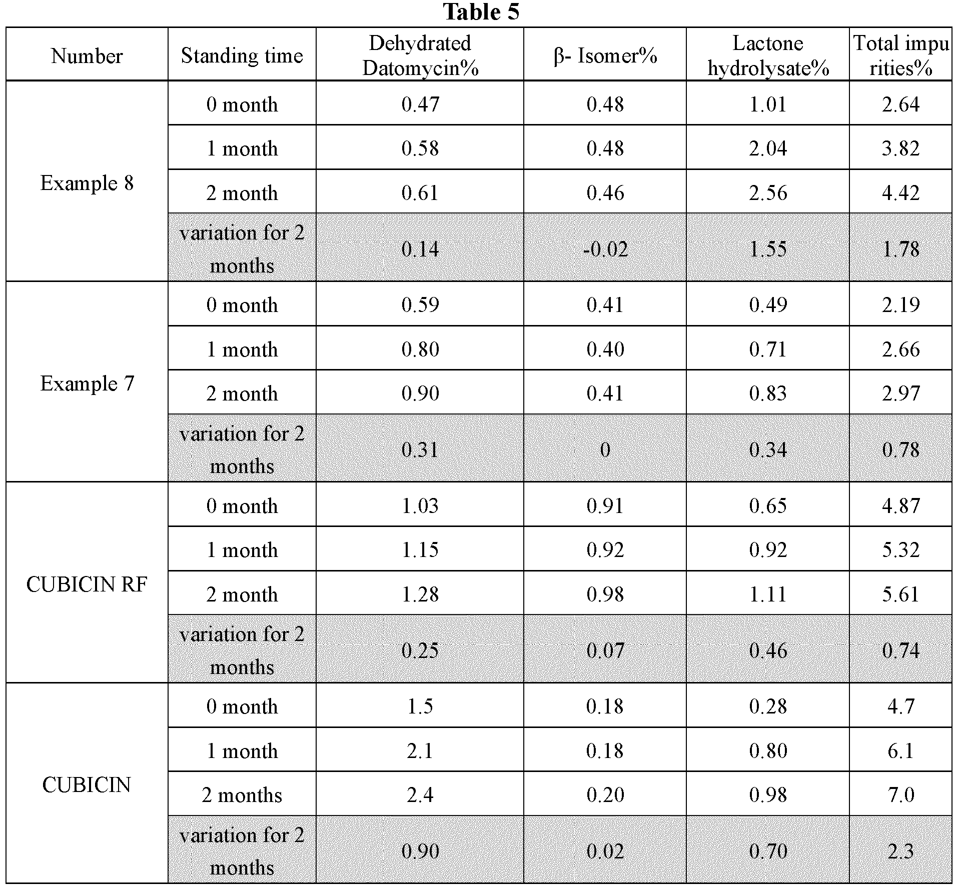

The results shows that the stability of spray drying powder prepared at pH5.0 is better than that of pH7.0 spray drying powders, is similar to that of CUBICIN RF, and is better than that of CUBICIN.

The stability of the weak acid sample (pH4.0-6.0) is better than that of the near neutral sample (pH6.0-7.0), after the solid powders of daptomycin is made by spray-drying.

### Example 9: Preparation of Datomycin Composition for Injection

Take 50.0g of daptomycin and 4.1g of calcium chloride, dissolve them in 250 mL of water for injection, then add sodium hydroxide solution to adjust pH 5.0, and then add 4.8g of sodium chloride, filter, and collect a filtrate, separately pack them into vials. After half stoppering, place the vials with the filtrate to a freeze-drying oven to pre-frozen for about 4 hours below -50°C. And then heat up under program control and control a temperature below -5°C, control a vacuum degree around 0.25 mbar, and control a sublimation drying for 20 hours. Dry the product for about 5 hours at a temperature not higher than 20°C by using a program temperature control. Fill with nitrogen, press a stopper, and then take out of the freeze-drying oven and roll a lid.

### Examples 10-14: Preparation of Datomycin Composition for Injection

The daptomycin composition for injection and its composition in Examples 10-14 are shown in Table 6. Preparation according to the method of Example 7: take 5 portions of datamycin, 50.0 g per portion, respectively take 6.0 g calcium chloride and 3.8 g sodium chloride, 8.6 g calcium chloride and 2.0 g sodium chloride, 12.0 g calcium chloride, 13.7 g calcium chloride, and 20.6 g calcium chloride, dissolve them to 250mL of water for injection, then add a sodium hydroxide solution to adjust to pH 5.0, filter, collect a filtrate, and freeze dry the filtrate to prepare a freeze-dried powder.

**Table 6**

| Number | Datomycin (g) | Calcium chloride (g) | Sodium chloride (g) | pH |
|---|---|---|---|---|
| Example 10 | 50 | 6 | 3.8 | 5.0 |
| Example 11 | 50 | 8.6 | 2 | 5.0 |
| Example 12 | 50 | 12 | -- | 5.0 |
| Example 13 | 50 | 13.7 | -- | 5.0 |
| Example 14 | 50 | 20.6 | -- | 5.0 |

### Test Example 6: Preparation stability with different calcium chloride content

Take the freeze-dried dry powder prepared by Examples 9-12, the original commercially available preparations CUBICIN RF and CUBICIN, and place them at 40°C for one month to investigate the changes in related substances. The results are shown in Table 7.

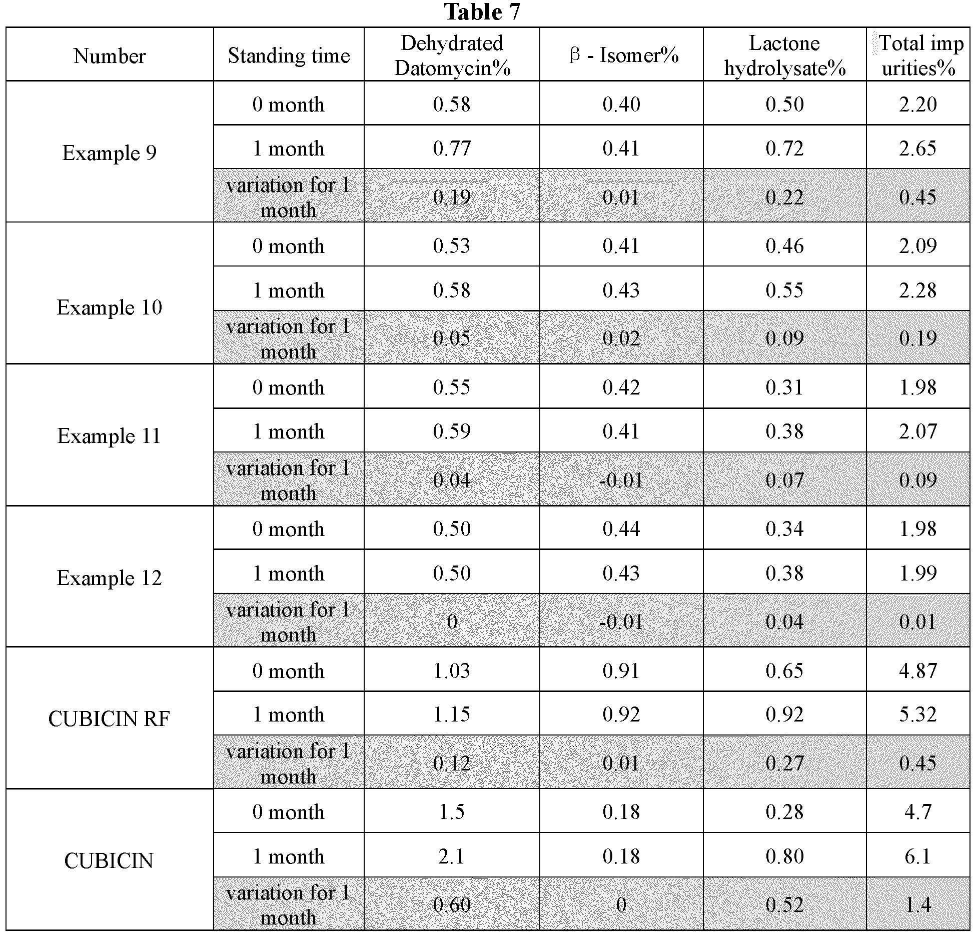

The results shows that as the proportion of calcium ions increases, the stability of freeze-drying powders increases.

### Test Example 7: Clarification of Datomycin Composition for Injection after Dissolution

Add the freeze-drying powders prepared by Example 9, Example 11, Example 12, Example 13, and Example 14 to water for injection, and dissolve to a 50mg/ml Daptomycin solution. The solution is compared with the turbidity standard solution according to the Chinese Pharmacopoeia 2020. The results are shown in Table 8.

**Table 8**

| Number | Datomicin: Calcium ion (molar ratio) | Clarity |
|---|---|---|
| Example 9 | 1:1.2 | Less than No. 1 turbidity standard solution |
| Example 11 | 1:2.5 | Near No. 1 turbidity standard solution |
| Example 12 | 1:3.5 | Near No. 1 turbidity standard solution |
| Example 13 | 1:4 | Less than No. 2 turbidity standard solution |
| Example 14 | 1:6 | Less than No. 2 turbidity standard solution |

The results shows that when the molar ratio of daptomycin to calcium ion is less than 1:2.5, the solution clarity is less than or close to No. 1 turbidity standard solution. When the molar ratio of daptomycin to calcium ion exceeds 1:3.5, the solution clarity is less than No. 2 turbidity standard solution. It has exceeded the turbidity standard solution No.1 and does not meet the clarity standard.

### Test Example 8: Study on Osmotic pressure of the Datomicin Composition for Injection

Take 5 parts of datomycin, each part has 500mg. Dissolve each part and 41 mg of calcium chloride, 85.7 mg of calcium chloride, 120 mg of calcium chloride, 137 mg of calcium chloride and 205.7 mg of calcium chloride to 10 ml of water for injection respectively, then add a sodium hydroxide solution to adjust to pH 5.5, filter, collect a filtrate, and then divide it into vials, place in a freeze drying oven after half stopper, the vials is pre frozen for about 4 hours at a temperature below -30°C, and then heat up under program control to control a temperature below -5°C, control a vacuum degree around 0.05mbar, and control a sublimation drying for 20 hours. Dry the product for about 5 hours at a temperature not higher than 20°C by using a program temperature control. Fill with nitrogen, press a stopper, and then take out of the freeze-drying oven and roll a lid. Add water for injection to dissolve it, so that the concentration of daptomycin is 50mg/ml. The osmotic pressure is determined according to the freezing point reduction method in "0632" of Chinese Pharmacopoeia 2020 (Part IV), and the results are shown in Table 9.

**Table 9**

| Datomicin: Calcium ion (molar ratio) | Osmotic pressure (mosm) |
|---|---|
| 1:1.2 | 150 |
| 1:2.5 | 220 |
| 1:3.5 | 300 |
| 1:4 | 365 |
| 1:6 | 530 |

The results shows that when the molar ratio of daptomycin to calcium ion is 1:3.5, the Osmotic pressure of molar concentration solution is about 300mosm, which is close to the osmotic pressure of human plasma and is an isotonic solution.

The above description of the specific embodiments of the present invention does not limit the present invention. Those skilled in the art can make various changes or modifications based on the present invention, as long as it does not deviate from the spirit of the present invention, it should fall within the scope of protection of the claims of the present invention.

## Claims

1. A high-stability datomicin composition for injection, wherein the datomicin composition comprises datomicin and a pharmaceutically acceptable carrier, the pharmaceutically acceptable carrier contains divalent metal salts, pH regulators and osmotic pressure regulators; wherein the molar ratio of daptomycin to the divalent metal salt in the daptomycin composition for injection is 1:1.0-1:3.5.

2. The datomicin composition for injection according to claim 1, wherein the divalent metal salt is selected from the group consisting of calcium nitrate, calcium chloride, calcium hydrobromate, calcium hydroiodate, calcium gluconate, calcium hydrogen phosphate, calcium lactate, calcium acetate, calcium phosphate, magnesium nitrate, magnesium chloride, magnesium hydrobromate, magnesium hydroiodate, magnesium sulfate, magnesium gluconate, magnesium hydrogen phosphate, magnesium lactate, magnesium acetate, magnesium phosphate, zinc nitrate, zinc chloride, zinc hydrobromate, zinc hydroiodate, zinc sulfate, zinc gluconate, zinc hydrogen phosphate, zinc lactate, zinc acetate, and zinc phosphate.

3. The datomicin composition for injection according to claim 1, wherein the pH regulator is selected from the group consisting of sodium hydroxide, calcium hydroxide, calcium lactate, sodium lactate, sodium phosphate, disodium hydrogen phosphate, sodium dihydrogen phosphate, potassium phosphate, dipotassium hydrogen phosphate, potassium dihydrogen phosphate, citric acid, sodium citrate, sodium bicarbonate, and sodium carbonate.

4. The datomicin composition for injection according to claim 1, wherein the osmotic pressure regulator is selected from the group consisting of sodium chloride, glucose, phosphate, and citrate.

5. The datomicin composition for injection according to claim 1, wherein the pH of the datomicin composition for injection is 3.5-6.0, preferably 4.0-5.5, more preferably 4.5-5.0.

6. The datomicin composition for injection according to claim 1 or 4, wherein the molar ratio of daptomycin to the osmotic pressure regulator is 1:0-3.0, preferably 1:0.5-2.75, more preferably 1:1.0-2.5, and further preferably 1:1.5-2.25.

7. The datomicin composition for injection according to claim 1, 2 or 4, wherein the molar ratio of daptomycin to the divalent metal salt to the osmotic pressure regulator in the datomicin composition for injection is 1:1.0-3.5:0-3.0, preferably 1:1.2-3.0:0.5-2.75, more preferably 1:1.5-2.75:1.0-2.5, and further preferably 1: 1.75-2.50:1.5-2.25.

8. The datomicin composition for injection according to claim 7, wherein the molar ratio of daptomycin to calcium chloride to sodium chloride in the datomicin composition for injection is 1:1.5:2.5 and to adjust the pH of the datomicin composition for injection to 4.5 by sodium hydroxide.

9. The datomicin composition for injection according to claim 1, wherein the total impurity content in the datomicin composition for injection is ≤ 7.0%, and the content of the dehydrated daptomycin is ≤ 4.0%, the content of the β- Isomer is ≤ 1.5%, the content of the lactone hydrolysate ≤ 1.5%.

10. A preparation method for a high-stability datomycin composition for injection according to any one of claims 1-9, wherein the datomycin composition for injection comprises datomycin and a pharmaceutically acceptable carrier, the pharmaceutically acceptable carrier contains divalent metal salts, pH regulators, and osmotic pressure regulators; wherein the molar ratio of daptomycin to the divalent metal salt in the daptomycin composition for injection is 1:1.0 to 1:3.5; the method comprises the following steps:
dissolving a required amount of daptomycin and a divalent metal salt in water for injection to form an aqueous solution, adding a pH regulator to the aqueous solution, and then adjusting a pH of the aqueous solution to 3.5-6.0, adding a required amount of the osmotic pressure regulator to adjust the aqueous solution to isotonic, filtering to obtain a filtrate , and then treating the filtrate by freeze-drying or spray-drying to obtain a datomycin composition for injection.

11. The preparation method according to claim 10, wherein the freeze-drying method comprises the following steps:
1) filling the filtrate into a penicillin bottle, and then placing it in a freeze-drying oven, cooling to -20°C~ -70°C to make the filtrate frozen completely;
2) heating up to -10°C-5°C, and then maintaining a vacuum of 0-0.25 mbar, and drying;
3) heating up to 5°C~40°C, and drying to obtain a datomycin composition.

12. The preparation method according to claim 10, wherein the spray-drying method comprises the following steps: setting an inlet air temperature to 150-230, an outlet air temperature to 80-150°C; after these parameters are stabilized, spray-drying an aqueous solution of daptomycin, to obtain a datomycin composition.

13. A use of the high-stability datomicin composition for injection in the preparation for antibacterial drugs, wherein the datomicin composition for injection is used to treat infections caused by gram positive bacteria, the infection is selected from the group consisting of skin and soft tissue infections, endocarditis, and bacteremia.
